# EUROPEAN PATENT APPLICATION

(11) **EP 3 488 891 A1**
(43) Date of publication of application: **29.05.2019**
(21) Application number: 18208116.6
(22) Date of filing: 23.11.2018
(51) Int. Cl.: A61M 25/01, A61B 1/00, B08B 9/049, A61B 34/30, A61M 25/10

(54) **DEVICE FOR TRANSLATIONAL MOVEMENT THROUGH VESSELS**

(30) Priority: 27.11.2017 US 201762590899 P
(71) Applicant: Cook Medical Technologies LLC, Bloomington, IN 47404 (US)
(72) Inventor: MERK, James C., Terre Haute, IN Indiana 47802 (US); MAYLE, Brent A., Spencer, IN Indiana 47460 (US); Neff, Gary, Bloomington, IN Indiana 47408 (US); Spindler, Ralf, Bloomington, IN Indiana 47401 (US)
(74) Representative: Williams Powell

(57) **Abstract**

A device for insertion into a vessel and suitable for implementing translational movement through the vessel (10) includes a flexible tube (12) containing a translational actuator. Anchoring actuators are positioned along the length of the flexible tube (12) and are configured for example to controllably expand against the inner wall of the vessel in coordination with extension and contraction of the translational actuator to effect translational movement.

## Description

The present disclosure relates generally to apparatuses and methods for treating vascular conditions, and more specifically, to apparatuses and methods for advancing a mechanism through a vessel.

### BACKGROUND

Atherosclerosis and other occlusive diseases are prevalent among a significant portion of the population. In such diseases, atherosclerotic plaque forms within the walls of the vessel and blocks or restricts blood flow through the vessel. Atherosclerosis commonly affects the coronary arteries, the aorta, the iliofemoral arteries and the carotid arteries. Several serious conditions may result from the restricted blood flow, such as ischemic events.

Various procedures are known for treating stenoses in the arterial vasculature, such as the use of balloon angioplasty and stenting. During a balloon angioplasty procedure, a catheter having a deflated balloon attached thereto is positioned across a constricting lesion, and the balloon then is inflated to widen the lumen to partially or fully restore patency to the vessel.

Stenting involves the insertion of a usually tubular member into a vessel, and may be used alone or in conjunction with an angioplasty procedure. Stents may be self-expanding or balloon expandable. Self-expanding stents typically are delivered into a vessel within a delivery sheath, which constrains the stent prior to deployment. When the delivery sheath is retracted, the stent is allowed to radially expand to its predetermined shape. If the stent is balloon expandable, the stent typically is loaded onto a balloon of a catheter, inserted into a vessel, and the balloon is inflated to radially expand the stent.

Generally, during each of the foregoing interventional procedures, a wire guide is inserted into a patient's vessel, e.g., under fluoroscopic guidance. The wire guide then is advanced toward a target site in a patient's vasculature. For example, the proximal end of the wire guide may be advanced through a stenosis. Then, various medical components, such as a balloon catheter and/or stent, may be proximally advanced over the wire guide to the target site.

Various wire guides comprise flexible proximal regions to facilitate navigation through the tortuous anatomy of a patient's vessel, but such flexible proximal regions may be difficult to be advanced through an occlusion. However, if the proximal region of the wire guide is too rigid, then it may not be flexible enough to navigate the tortuous anatomy. It may be difficult to advance both relatively flexible and relatively rigid wire guides through an occlusion, particularly a narrow or hardened stenosis, by manually pushing from the distal end of the wire guide.

Because the control of movement and placement of medical devices within the body using wire guides relies on mechanical forces that are externally applied, the forces may not be accurately controlled and may potentially cause damage to a vessel wall. Additionally, an open access port for controlling a device may increase a chance of infection.

### BRIEF SUMMARY

In order to further reduce the chance of damaging a vessel wall when moving a medical device in a vessel, a motorized apparatus and method for generating translational movement through a vessel without requiring a guidewire is described. The motorized apparatus may be powered by cyclic movement of fluids and/or powered electrically. The motorized apparatus may be wired or wireless and may transport medical devices, sensors or medications for diagnostic and/or therapeutic use.

According to one aspect, an apparatus suitable for translational movement through an interior of a vessel is described. The apparatus may include a flexible tube having a first end and a second end. A first vessel wall grappling member may be positioned on an exterior of the flexible tube adjacent the first end and a second vessel wall grappling member may be positioned on the exterior of the flexible tube adjacent the second end. The first and second wall grappling members are adapted to selectively expand to contact a vessel wall surrounding the flexible tube. An actuation mechanism may be positioned along at least a portion of a length of the flexible tube, where the actuation mechanism is configured to cooperate with the first and second vessel wall grappling members to effect movement of the flexible tube in at least one direction relative to the vessel wall.

According to another aspect, a method for moving a device through a passageway is provided. The method includes inserting the device into the passageway, the device having a hollow flexible tube with a first wall gripping member and a second wall gripping member, where each of the first and second wall gripping members are positioned at opposite ends of an outside portion of the hollow flexible tube. The method may further include expanding the first wall gripping member to an expanded state on a first end of the hollow flexible tube and extending the hollow flexible tube to an extended position while maintaining the first wall gripping member in the expanded state. After extending the hollow flexible tube, the method may include expanding a second wall gripping member to the expanded state on a second end of the hollow flexible tube and contracting the first wall gripping member to a contracted state. The method further includes retracting the hollow flexible tube to a retracted position while the second wall gripping device is in the expanded state and while the first wall gripping device is in the contracted state.

In yet another aspect, an apparatus is disclosed that is suitable for translational movement through an interior of a vessel. The apparatus may have a flexible body having a first end and a second end opposite the first end. A first wall contact actuator may be positioned on an exterior of the flexible body at the first end and configured to selectively contact the interior of the vessel. A second wall contact actuator may be positioned on the exterior of the flexible body at the second end, where the second wall contact actuator is configured to selectively contact the interior of the vessel. A body actuator may be mounted to the flexible body between the first and second wall contact actuators, the actuator configured to extend and contract a length of the flexible body. The first and second wall contact actuators and the body actuator are adapted for independent actuation to selectively move the flexible body along a longitudinal axis of the vessel.

Other systems, methods features and advantages will be, or will become, apparent to one of skill in the art upon examination of the following figures and detailed description. It is intended that all such additional systems, methods, features and advantages be within the scope of the invention, and be encompassed by the following claims.

### BRIEF DESCRIPTION OF THE DRAWINGS

Embodiments of the invention are described below by way of example only, with reference to the accompanying drawings.
FIG. 1 is a perspective view of one embodiment of an apparatus for moving through a vessel.
FIGS. 2A-2F illustrate a sequence of actuator actions on the apparatus of FIG. 1 for causing translational movement of the apparatus along a vessel.
FIG. 3A illustrates an end view of another anchoring actuator usable in the apparatus of FIG. 1 in an inflated state.
FIG. 3B illustrates the anchoring actuator of FIG. 3A in a deflated state.
FIG. 4A illustrates an end view of another anchoring actuator usable in the apparatus of FIG. 1 in an expanded state.
FIG. 4B illustrates the anchoring actuator of FIG. 4A in a retracted state.
FIG. 5A illustrates the apparatus of FIG. 1 approaching a stenosis in a vessel.
FIG. 5B illustrates the apparatus of FIG. 1 moving into a stenosis in a vessel.

### DESCRIPTION OF THE PREFERRED EMBODIMENTS

In the present application, the term "distal" refers to a direction that is generally toward a physician during a medical procedure, while the term "proximal" refers to a direction that is generally toward a target site within a patient's anatomy during a medical procedure.

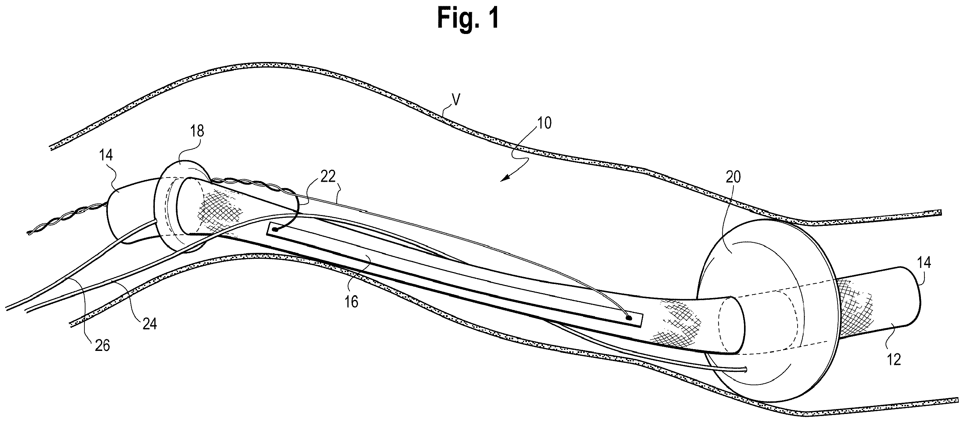

Referring now to FIG. 1, an apparatus 10 suitable for advancing along the interior of a vessel V is shown and described. The apparatus 10 may include a flexible body in the form of a tube 12 defining a passageway terminating at open ends 14. The flexible tube 12 may be formed from one or more semi-rigid polymers. For example, the flexible tube 12 may be manufactured from polyurethane, polyethylene, tetrafluoroethylene, polytetrafluoroethylene, fluorinated ethylene propylene, nylon, PEBAX or the like. Other suitable materials may include silicone and shape memory polymers. The apparatus 10 may include a translational actuator 16, a first anchoring actuator 18 and a second anchoring actuator 20.

The translational actuator 16, also referred to herein as a body actuator, may be positioned on at least a portion of an exterior of the flexible tube 12. In other implementations, the translational actuator 16 may be positioned in the flexible tube 12 or may form part of the flexible tube 12. The flexible tube 12 may comprise a corrugated or bellows-like surface, in one implementation, to help the flexible tube 12 expand and contract along its longitudinal axis in cooperation with the translational actuator 16. Each of the first and second anchoring actuators 18, 20, also referred to herein as vessel wall grappling or gripping members, may be positioned on an outside of the flexible tube 12 and spaced apart from one another. In one implementation, as shown in FIG. 1, the first and second anchoring actuators 18, 20, may circumferentially surround, and be positioned at respective opposite ends of, the flexible tube 12 adjacent the openings 14.

The translational actuator 16 may be configured to change in length from a first, minimal length state to a second, extended length state along a longitudinal axis of the flexible tube 12 in response to receipt of a remotely controlled input. The first and second actuators 18, 20 may each be configured to expand and contact an interior wall of a surrounding vessel V in a first state, as well as to contract and pull away from the interior wall of vessel V in a second state, in response to remotely controlled input. The translational actuator 16 and first and second actuators 18, 20 may all be independently actuated. As described in greater detail below, the translational actuator 16 and first and second anchoring actuators 18, 20 are configured to cooperate to effect translational movement of the apparatus 10 in either the proximal or distal direction along a vessel V without the need for first inserting a guide wire and without using a wire to mechanically push or pull the apparatus along the vessel.

In FIG. 1, the translational actuator 16 is illustrated as an electrically sensitive length of material, where the length of the material is changeable in response to an applied current or voltage. One suitable material for use as the electrically sensitive material for the translational actuator 16 is nitinol. Any of a number of shapes may be used for the translational actuator material. For example, the material may be formed into a helical spring shape that extends along the inside or the outside of the flexible tube 12, a straight wire that is attached to one side of the tube 12, or even two or more separately controllable wires positioned on different sides of the inside or outside of the flexible tube that may be controlled to both effect translation and steering of the flexible tube 12 via differing the amount of length changes at each wire. In one implementation, the flexible body 12 may be a spring-shaped body.

The translational actuator 16 may be remotely controlled via one or more insulated input wires 22 that attach to the translational actuator 16 and may extend from the apparatus 10 through the vessel V to a voltage or current source (not shown) outside an entry point at a distal region of the vessel V. The translational actuator 16 may have its length changed from an initial, minimal length state, where no electrical stimulus is applied and the length of the actuator is at its minimum, to an extended length state, where electrical stimulus is applied such that the length of the actuator extends to an extended state where the length is greater that the length at the initial state. The change of length between states may be a fixed change of length, using a predetermined fixed voltage or current change, or may be variable over a predetermined range.

The example first and second anchoring actuators 18, 20 shown in FIG. 1 may be inflatable toroidal (donut-shaped) balloons that are positioned to expand outwardly from the flexible tube 12 or contract to an initial position in response to a fluid supplied by respective fluid ports 24, 26 that each connect to a respective one of the first and second anchoring actuators 18, 20. The fluid ports 24, 26 may also extend in parallel with the input wires 22 to the same entry point at the distal region of the vessel V where they are may be connected to a fluid source (not shown) that may inject and remove a fluid into the respective anchoring actuators to control expansion and contraction. The fluid may be a liquid or a gas. The size of the toroidal balloons, and therefore the degree of friction against the vessel wall provided by either of the anchoring actuators, may be controlled by changing the pressure of fluid applied via the respective fluid ports 24, 26.

Although specific actuator mechanisms, electrical and fluid, are shown in the example of FIG. 1, it is contemplated that different types of actuator mechanisms, in different combinations, may be utilized. As one example, all of the actuators 16, 18, 20 may be piezoelectric actuators, where the dimensional changes of the anchoring actuators 18, 20 may be achieved with electrical, rather than fluid pressure, in the a similar manner as described with the translational actuator 16 of FIG. 1. Another example of a suitable translational actuator 16 may be a linear motor that works to lengthen or shorten a mechanically extendable structure, which may be any of a number of arrangements such as a drawer slide arrangement or multiple section telescoping arrangement, where each different mechanical component may be attached to the flexible tube 12. The motor portion of this translational actuator may comprise an Inchworm ® motor manufactured by EXFO Burleigh Products Group, Inc. of Victor, N.Y. A variable-rate staircase voltage may be applied to the motor, causing it to change length in discrete steps on the order of nanometers or other desired increment. In yet other implementations, it is also contemplated that a wireless version of the apparatus 10 of FIG. 1 may be implemented, where external control via wires and/or fluid ports may be avoided and input control stimulus to the apparatus 10 provided in a wireless manner, such as by inductive transmission of electrical power and stimulus. In other embodiments, the electrically driven portions may be accomplished wirelessly with only the need to fluid ports to be provided from a remote fluid source outside the vessel V. In yet other embodiments, the apparatus 10 may carry a battery or other capacitive element (not shown) to power one or more of the actuators. Such a battery powered version may respond to external triggers, for example signals transmitted to the apparatus 10 via magnetic induction, to trigger transition between states for one or more of the actuators.

Regardless of the particular configuration of actuators in the apparatus 10, it is contemplated that the apparatus may be injected, endovascularly delivered or otherwise surgically placed inside a vessel in a patient's body at an initial location and remotely controlled by electrical and/or fluid control to move itself to a desired location along the vessel V from that initial location.

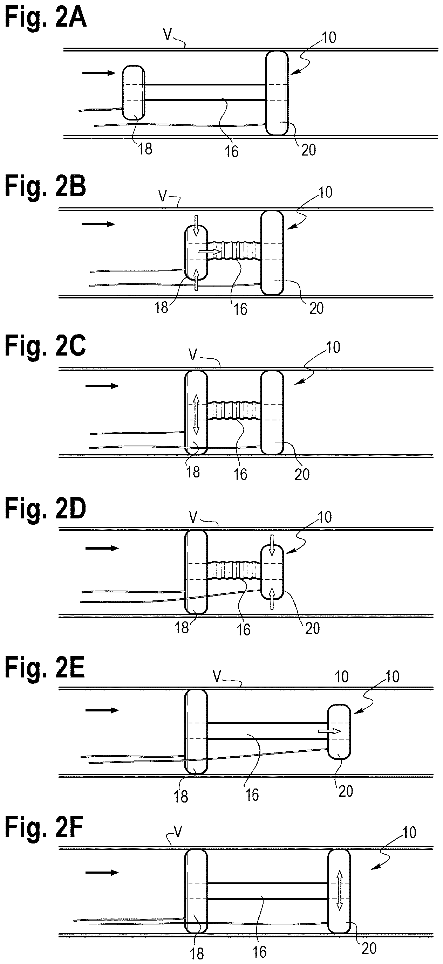

Referring now to FIGS. 2A-2F, a simplified cross-sectional schematic of the apparatus 10 of FIG. 1 is shown to illustrate one example of how the actuators on the apparatus may cooperate to produce a worm-like movement that will move the apparatus 10 along the interior of a vessel V. The anchoring actuators 18, 20 and translational actuator 16 each have first states, in which they are relaxed, and second states, in which they are actuated. Each of the actuators 16, 18, 20 may be operated independently to be transformed from the first state to the second state by selectively applying and removing an electric signal (translational actuator example of FIG. 1) or a fluid change (anchoring actuators 18, 20 of FIG. 1) to each of the actuators, as explained below.

In FIG. 2A, the first anchoring actuator 18 is shown in its first, relaxed state, while the second anchoring actuator 20 and translational actuator 16 are shown in their actuated states, where the second anchoring actuator 20 has expanded to contact a vessel wall due to received fluid pressure and the translational actuator is at its extended length in response to an applied electric signal from an electric power source.

Referring now to FIG. 2B, in a first step for advancing the apparatus 10 proximally with respect to a target position along the vessel V, the translational actuator 16 may be switched to its first state, where its length is at a minimum, by removing the electric signal previously applied to it. When this is done while only the second anchoring actuator 20 is contacting the vessel wall and the first anchoring actuator is in its first, relaxed state, then the reduction of length of the translational actuator 16 pulls the end of the apparatus 10 with the first anchoring actuator 18 toward the second anchoring actuator in a proximal direction.

In a next step, shown in FIG. 2C, the first anchoring actuator 18 may be actuated to expand and contact the vessel V and form a friction fit against the interior wall of the vessel V. More specifically, the first anchoring actuator 18 may be actuated by applying a fluid pressure via a fluid port, thereby increasing the size of the first anchoring actuator 18, as shown in FIG. 2C.

Referring now to FIG. 2D, with the first anchoring actuator 18 in its actuated state, the second anchoring actuator 20 is then reduced in size to its first, relaxed state. The second anchoring actuator 20 may be reduced in size by removing fluid via the fluid port which causes the second anchoring actuator to reduce in diameter and pull away from the vessel V. Subsequently, an electric signal may be applied to the translational actuator 16 to cause the translational actuator 16 to extend to its second, extended length. This step is shown in FIG. 2E and shows how, while maintaining actuation of the first anchoring actuator 18 such that only the first anchoring actuator engages in a frictional manner with the vessel wall where the second anchoring actuator 20 is in its relaxed state such that it does not engage the vessel wall, the end of the apparatus with the second anchoring actuator is moved proximally along the vessel V. FIG. 2F then illustrates the last of the sequence of steps, where the second anchoring actuator is again actuated to expand and contact the vessel to maintain the position now reached along the vessel by the apparatus 10. The first anchoring actuator may then be relaxed by removing fluid from the first anchoring actuator 18 to collapse it and retract it from the inner wall of the vessel. This returns the apparatus to the configuration of FIG. 2A, but now at a proximally advanced position from the start of the sequence. At this point, the series of actuation steps between FIGS. 2B-2F may be repeated to continue to advance the apparatus 10 in a proximal direction.

The process described in FIGS. 2A-2F may be repeated as frequently as desired. For example, the process may be repeated many times per second. If piezoelectric actuators are employed for all of the actuators, they may be operated over many cycles without significant wear or deterioration. If the first, second and third actuators 16, 18, 20 comprise piezoelectric actuators, then incremental positioning may be obtained on a scale of nanometers. Accordingly, it may be possible to move the apparatus on a nanometer scale over several millimeters of continuous motion. The configuration and type of the actuators 16, 18, 20 on the apparatus 10 may enable higher resolution positioning of the apparatus than, for example, pushing the apparatus manually via wire guide.

Moreover, the apparatus 10 may be moved incrementally in a distal direction by reversing the steps described for FIGS. 2A-2F. For example, assuming a current orientation of the actuators 16, 18, 20 of the apparatus 10 is represented by FIG. 2F, in a first step to begin distal movement of the apparatus 10, the second anchoring actuator 20 may be reduced in size to retract away from the vessel V as shown in FIG. 2E. Then, while the second anchoring actuator 20 is in the relaxed state and the first anchoring actuator is in the actuated state and forms a friction fit against the wall of the vessel V, the translational actuator 16 may be relaxed to its minimum length to move the apparatus 10 in a distal direction. Similarly, the rest of steps in FIGS. 2A-2D may be implemented in reverse sequence to continue the movement of the apparatus in the distal direction.

As noted above, the translational actuator 16 may be a piezoelectric actuator, which therefore undergoes a dimensional change when an electrical signal is applied. The dimensional change may be proportional to the voltage or current applied to the actuator. Accordingly, the provision of a variable voltage to the translational actuator 16 may impact the linear change associated with the translational actuator 16 and may affect the incremental linear movement of the apparatus.

In one implementation, the flexible tube 12 of the apparatus 10 is configured to provide a continuous unobstructed passage between the proximal and distal ends of the flexible tube 12 for a fluid to pass. In other words, the flexible tube 12 may provide passage for fluids at all actuation stages of the first and second anchoring actuators 18, 20, and of the translational actuator 16, shown in FIGS 2A-2F. This open passage through the flexible tube 12 may allow for fluids to move through the vessel while one or both of the anchoring actuators are actuated and have expanded to press against the inside wall of the vessel V. In implementations (See FIG. 1) where the first and second anchoring actuators are toroidal-shaped balloons that completely encircle the outer circumference of the flexible tube 12, fluid flow may be completely blocked around the outside of the flexible tube, but the passageway defined through the flexible tube 12 permits fluid to flow through the vessel. The first and second anchoring actuators 18, 20 may be configured to only expand outwardly from the flexible tube 12 in response to actuation to avoid pinching off the passageway defined through the flexible tube 12.

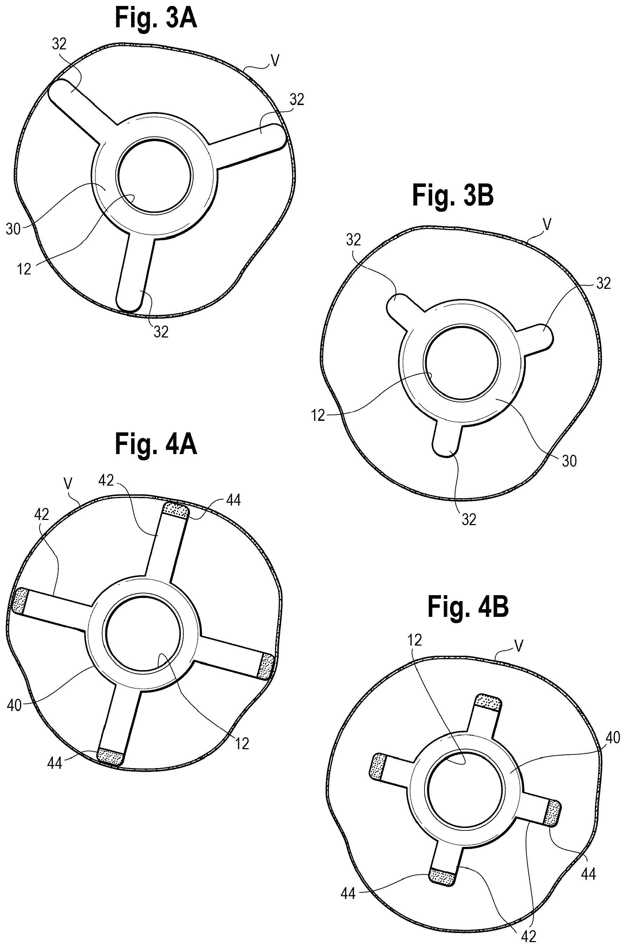

In other implementations, the first and second anchoring actuators 18, 20 may be inflatable balloons that surround the flexible tube 12 but that do not completely block off fluid from passing outside the flexible tube 12 when actuated. For example, a version of an anchoring actuator 30 is shown in FIG. 3A (inflated) and FIG. 3B (deflated) in the form of a balloon with circumferentially spaced inflatable portions 32 in staggered positions around the flexible tube 12 that may be used rather than a continuous toroidal balloon. In this arrangement of an anchoring actuator 30, some fluid in the vessel V may also pass between the inflated columns 32 of the anchoring actuator 30 and around the outside of the flexible tube. In yet other implementations of the anchoring actuators, piezoelectric material, such as nitinol, or other electrically controllable braces or anchoring members may be used instead of balloons. As shown in FIGS. 4A-4B, these electrically operable actuators may be spaced around the circumference of the flexible tube 12 to expand against and retract from the inner wall of a surrounding vessel in which the apparatus 10 is inserted. The example anchoring actuator 40 of FIGS. 4A-4B includes a plurality of extendible legs 42, which may have a gripping material such as a rubberized foot 44 coating it or affixed to the ends, may expand from (FIG. 4A) and retract against (FIG. 4B) the outer circumference of the flexible tube 12 in the same sequence illustrated in FIGS. 2A-2F, to move the apparatus through a vessel V. It is contemplated that a piezoelectric version of the anchoring actuators would include legs 42 having spacing between them to also allow fluid to flow outside of the flexible tube 12 as illustrated in FIGS. 4A-4B. Instead of rubberized feet 44, other gripping materials, including but not limited to barbed members, or a split cannula, may be used with the legs 42 to improve traction against the vessel. Also, any of a number of other anchoring actuator configurations that allow a remotely provided electrical or fluid force may be utilized in the apparatus 10.

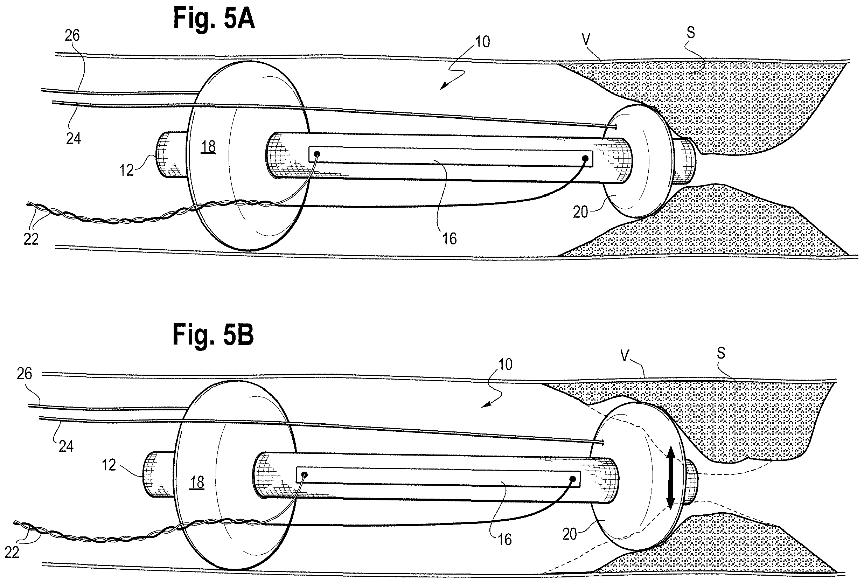

The apparatus 10 may be used to treat a stenosis in the vessel or used to pull or position medical instruments, tools or even medications to a desired location in a vessel. The apparatus 10 may be inserted into the patient's vessel V and positioned proximal to a stenosis S in the manner described above with respect to FIGS. 2A-2F by inserting the apparatus 10 into a vessel V at a distal location and causing the apparatus to traverse the vessel V in a proximal direction to the stenosis S. When the leading end of the apparatus reaches the stenosis S (FIG. 5A), the second actuator 20 may then be inflated (FIG. 5B to push against and widen a passage through the stenosis. In other embodiments, the apparatus 10 may be used to pull into place against the stenosis a separate device (not shown), such as a drug delivery system, camera system, spectroscopic system, or biopsy system, that may be directed through the flexible tube 12 into the stenosis S for appropriate diagnostics and treatment of the stenosis.

It will be apparent that while the embodiments have been described primarily with respect to advancing an apparatus having inflatable anchoring actuators and an electrically controllable translation actuator through a vessel. In various implementations, the apparatus is designed to, in an inch worm-like manner, propel itself through a vessel using fluid and/or electrical energy provided from remotely located sources. The remotely located sources of electrical energy and/or fluids may be linked to the apparatus via wires and fluid bearing tubes that extend to the entry point in the vessel. In instances where electrical energy is provided to the apparatus, the electrical energy may be provided wirelessly via an inductive energy transmitting device positioned outside of the vessel and or body of the patient.

By using adjustable actuators that grip the inside of the vessel walls, no guidewire insertion is needed and the navigation of the vessel may be made by the apparatus itself rather than being propelled by a mechanical force of a guide wire or by pulling along a guide wire previously manually threaded through a vessel. The use of a relatively autonomous vessel navigating apparatus such as described herein may contribute to shorter insertion times in certain applications as a separate initial guide wire insertion procedure is not required.

While various embodiments of the invention have been described, it will be apparent to those of ordinary skill in the art that many more embodiments and implementations are possible within the scope of the invention. Accordingly, the invention is not to be restricted except in light of the attached claims and their equivalents. Moreover, the advantages described herein are not necessarily the only advantages of embodiments of the invention and it is not necessarily expected that every embodiment of the invention will achieve all of the advantages described.

The foregoing description of embodiments of the inventions has been presented for purposes of illustration and description, and is not intended to be exhaustive or to limit the inventions to the precise forms disclosed. It will be apparent to those skilled in the art that the present inventions are susceptible of many variations and modifications coming within the scope of the following claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application no. 62/590,899, from which this application claims priority, and in the abstract accompanying this application, are incorporated herein by reference.

## Claims

1. An apparatus suitable for translational movement through an interior of a vessel, the apparatus comprising:
a flexible tube having a first end and a second end;
a first vessel wall grappling member positioned on an exterior of the flexible tube adjacent the first end;
a second vessel wall grappling member positioned on the exterior of the flexible tube adjacent the second end;
wherein the first and second wall grappling members are configured to selectively expand to contact a vessel wall surrounding the flexible tube; and
an actuation mechanism positioned along at least a portion of a length of the flexible tube, the actuation mechanism being configured to cooperate with the first and second vessel wall grappling members to effect movement of the flexible tube in at least one direction relative to the vessel wall.

2. The apparatus of claim 1, wherein the flexible tube defines a hollow passage extending from the first end to the second end.

3. The apparatus of any preceding claim, wherein the first vessel wall grappling member is configured to expand in response to an increase in a first fluid pressure, and to contract in response to a decrease in the first fluid pressure.

4. The apparatus of any preceding claim, wherein the first vessel wall grappling member comprises a balloon.

5. The apparatus of any preceding claim, wherein the first vessel wall grappling member comprises a toroidal shape.

6. The apparatus of any preceding claim, wherein the actuation mechanism is configured to repeatedly extend the flexible tube to a first length along a longitudinal axis of the flexible tube and contract the flexible tube to a second length shorter than the first length along the longitudinal axis.

7. The apparatus of any preceding claim, wherein the actuation mechanism comprises an electrically controllable mechanism.

8. The apparatus of claim 7, wherein the electrically controllable mechanism is formed of a material configured to extend in length in response to receiving a first voltage, and is configured to reduce in length in response to receiving a second voltage different than the first voltage.

9. The apparatus of any preceding claim, wherein the first vessel wall grappling member comprises a material configured to adjustably maintain contact with an interior of the vessel wall via a friction force.

10. A method for moving a device through a passageway comprising:
inserting the device into the passageway, the device having a hollow flexible tube with a first wall gripping member and a second wall gripping member, each of the first and second wall gripping members positioned at opposite ends of an outside portion of the hollow flexible tube;
expanding the first wall gripping member to an expanded state on a first end of the hollow flexible tube;
extending the hollow flexible tube to an extended position while maintaining the first wall gripping member in the expanded state;
after extending the hollow flexible tube, expanding a second wall gripping member to the expanded state on a second end of the hollow flexible tube and contracting the first wall gripping member to a contracted state; and
retracting the hollow flexible tube to a retracted position while the second wall gripping device is in the expanded state and while the first wall gripping device is in the contracted state.

11. The method of claim 10, wherein the passageway comprises a patient's vessel and expanding the first wall gripping member comprises introducing a fluid into the first wall gripping member.

12. The method of claim 10 or 11, wherein contracting the first wall gripping member comprises removing the fluid from the first wall gripping member.

13. The method of any of claims 10 to 12, wherein extending the hollow flexible tube comprises activating an actuation mechanism mounted to the hollow flexible tube to extend a length of the hollow flexible tube.

14. The method of any of claims 10 to 13, wherein retracting the hollow flexible tube comprises activating the actuation mechanism mounted to the hollow flexible tube to reduce the length of the hollow flexible tube.

15. The method of any of claims 10 to 14, wherein activating the actuation mechanism to extend the length comprises applying a first voltage to the actuation mechanism and activating the actuation mechanism to reduce the length comprises applying a second voltage to the actuation mechanism that differs from the first voltage.
